# EUROPEAN PATENT SPECIFICATION

(11) **EP 2 609 065 B1**
(45) Date of publication and mention of the grant of the patent: **08.07.2015**
(21) Application number: 11749638.0
(22) Date of filing: 12.08.2011
(51) Int. Cl.: C07C 41/09

(54) **PROCESSES FOR PRODUCING 1-BROMO-2 (CYCLOPROPYL METHOXY)-5 FLUORO-4- METHOXYBENZENE**
VERFAHREN ZUR HERSTELLUNG VON 1-BROM-2 (CYCLOPROPYL METHOXY-)5 FLUOR-4-METHOXYBENZEN
PROCÉDÉS DE PRODUCTION DE 1-BROMO-2 (CYCLOPROPYL MÉTHOXY)-5 FLUORO-4- MÉTHOXYBENZÈNE

(30) Priority: 26.08.2010 US 377267 P
(43) Date of publication of application: 03.07.2013
(73) Proprietor: Albemarle Corporation, Baton Rouge, LA 70801-1765 (US)
(72) Inventor: GIARD, Thierry, J., B-1300 Wavre (BE); MUTTERER, Vincent, L., B-1470 Bousval (BE); DURVAUX, Christophe, B-1050 Bruxelles (BE)
(74) Representative: UEXKÜLL & STOLBERG
(86) International application number: PCT/US2011/047521
(87) International publication number: WO 2012/027134

(56) References cited:
- WO-A1-2009/106531
- BELANGER, PATRICE C. ET AL: "Facile preparations of 4-fluororesorcinol", CANADIAN JOURNAL OF CHEMISTRY , 66(6), 1479-82 CODEN: CJCHAG; ISSN: 0008-4042, 1988, XP002663157,

## Description

### BACKGROUND

1-Bromo-2- (cyclopropyl methoxy)-5-fluoro-4-methoxybenzene is useful in the preparation of APIs for use in the pharmaceutical industry.

WO 2009/106531 A1 discusses the synthesis of 1-bromo-2- (cyclopropyl methoxy)-5-fluoro-4-methoxybenzene through the bromination of 3-methoxy-4-fluorophenol followed by alkylation in the presence of 1-bromomethyl cyclopropane. See, e.g., WO 2009/106531 A1 p. 185. Regarding 3-methoxy-4-fluorophenol, its preparation from resorcinol derivatives, using Belanger's procedure, is mentioned. See, e.g., Belanger, P. C. et al. Can J. Chem. 1988, 66, 1479-1482. The published route, starting from resorcinol or dimethyl resorcinol, is fraught with regioselectivity issues.

It would be beneficial to the chemical industry to have improved processes for producing 1-bromo-2- (cyclopropyl methoxy)-5-fluoro-4-methoxybenzene that overcome the regioselectivity issues of current processes and reduce significantly the number of steps.

### THE INVENTION

This invention meets the above-described needs by providing new processes for synthesis of both 4-fluoro-3-methoxyphenol (FMP) and 1-bromo-2- (cyclopropyl methoxy)-5-fluoro-4-methoxybenzene ("BCPMFMB").

This invention provides processes comprising: reacting 3, 4-difluorophenol in the presence of benzylbromide and potassium carbonate in 2-butanone, thereby yielding 4-(benzyloxy)-1',2-difluorobenzene; introducing methoxy moiety to 4-(benzyloxy)-1,2-difluorobenzene using a toluene/NMP mixture in the presence of potassium methoxide generated in situ from potassium tert-butoxide and methanol, thereby yielding 4-(benzyloxy)-1-fluoro-2-methoxybenzene; debenzylating 4-(benzyloxy)-1-fluoro-2-methoxybenzene under hydrogen pressure in the presence of palladium on charcoal; and distilling out 4-fluoro-3-methoxyphenol. This invention also provides such processes that further comprise O-alkylating the 4-fluoro-3-methoxyphenol with 1-bromomethyl cyclopropane, thereby yielding 3-methoxy-4-fluoro-1-cyclopropyl methoxybenzene; brominating the 3-methoxy-4-fluoro-1-cyclopropyl methoxybenzene in the presence of 1, 3-dibromo-5, 5-dimethyl hydantoin or N-bromo succinimide, thereby yielding 1-bromo-2-(cyclopropyl methoxy)-5-fluoro-4-methoxybenzene. The O-alkylating reaction can be done in 2-butanone in 99 % yield.

This invention also provides processes that comprise: alkylating 3,4-difluorophenol, thereby yielding an O-alkylated phenol derivative; reacting the yielded O-alkylated phenol derivative with potassium methoxide in a toluene/NMP mixture, thereby yielding 3-methoxy-4-fluoro-1-cyclopropyl methoxybenzene; brominating the 3-methoxy-4-fluoro-1-cyclopropyl methoxybenzene in the presence of 1, 3-dibromo-5, 5-dimethylhydantoin or N-bromo succinimide; and crystallizing 1-bromo-2-(cyclopropyl methoxy)-5-fluoro-4-methoxybenzene from isopropyl alcohol or ethanol. Also provided are such processes wherein the alkylating of 3,4-difluorophenol comprises alkylating the 3,4-difluorophenol with 1-bromomethyl cyclopropane, and wherein the alkylating of 3,4-difluorophenol comprises alkylating the 3,4-difluorophenol with 1-chloromethyl cyclopropane, and wherein the toluene/NMP mixture is in about a 4:1 v/v mixture.

This invention provides processes for the preparation of FMP and BCPMFMB from 3,4-difluorophenol. Processes of this invention can provide at least about a 72 % yield of FMP. This invention comprise reacting 3, 4-difluorophenol in the presence of benzylbromide and potassium carbonate in 2-butanone, which can provide about a 97 % crude yield of 4-(benzyloxy)-1,2-difluorobenzene; introducing methoxy moiety to 4-(benzyloxy)-1,2-difluorobenzene using a toluene/NMP mixture in the presence of potassium methoxide generated in situ from potassium tert-butoxide and methanol, which can be done at about 90 °C in an 8:2 v/v toluene/NMP mixture and can provide about a 97 % crude yield of 4-(benzyloxy)-1-fluoro-2-methoxybenzene; debenzylating 4-(benzyloxy)-1-fluoro-2-methoxybenzene under hydrogen pressure in the presence of palladium on charcoal; and distilling out 4-fluoro-3-methoxyphenol (FMP) in 72% overall yield. A representative synthetic scheme for this process is presented below:

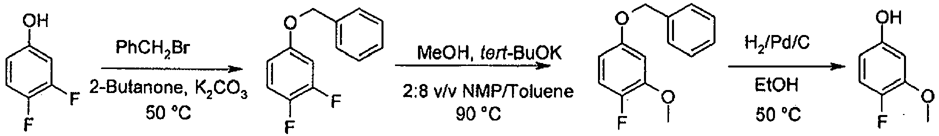

Thereafter, such processes according to this invention can continue with O-alkylating the 4-fluoro-3-methoxyphenol with 1-bromomethyl cyclopropane, thereby yielding 1-bromo-2-(cyclopropyl methoxy)-5-fluoro-4-methoxybenzene.

In processes of this invention, swapping of a benzyl group with a methyl cyclopropyl group is conducted. After alkylation of 3, 4 difluorophenol with 1-bromomethyl cyclopropane, which is performed in about 85% or greater yield under the reflux of water/2-butanone biphasic mixture or at 95 °C in a 66:33 to 8:2 vol/vol toluene/NMP mixture in the presence of sodium methoxide or potassium tert-butoxide as base, S_{N}Ar reaction is run. We have carried out the S_{N}Ar reaction successfully in 8:2 vol/vol toluene/NMP mixture in 97 % yield. Subsequently, the bromination step is performed. We have performed the bromination step in THF or in a mixture of toluene and THF in the presence of 1, 3-dibromo-5, 5-dimethylhydantoin to provide BCPMFMB in 95% crude yield. The BCPMFMB in final, desired form is then purified through recrystallization with isopropyl alcohol. We have finally purified BCPMFMB through a recrystallization from isopropyl alcohol in 92 % yield. A representative synthetic scheme for processes according to this invention is presented below:

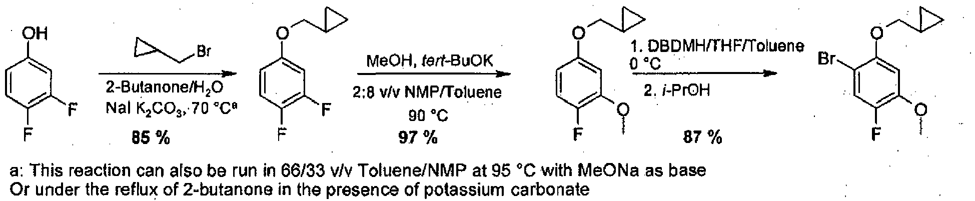

### EXAMPLES

The following examples are illustrative of the principles of this invention. It is understood that this invention is not limited to any one specific embodiment exemplified herein, whether in the examples or the remainder of this patent application.

### EXAMPLE 1.

(i) At ambient temperature, 2-butanone (500 ml) and potassium carbonate (108.22 g) were loaded into a flask.
**(ii)** The mixture was heated at 50 °C.
**(iii)** 3, 4-difluorophenol (100 g) diluted in 2-butanone (100 ml) was slowly added.
**(iv)** The mixture was stirred for 15 min.
**(v)** Benzyl bromide (134.05 g) was added.
**(vi)** The mixture was heated under reflux for 3 h.
**(vii)** The mixture was cooled down to ambient temperature.
**(viii)** Water (372 ml) was added.
**(ix)** The mixture was stirred and a phase cut (organic phase up) was conducted.
**(x)** The organic layer was washed with water (100 ml) and a phase cut (organic phase up) was conducted.
**(xi)** 2-butanone was distilled from the organic layer and toluene (175 ml) and NMP (43.75 ml) were added **(Mixture A).**
**(xii)** Toluene (350 ml) and NMP (87.5 ml) were loaded into a reactor.
**(xiii)** This mixture was stirred and heated at 40 °C.
**(xiv)** Methanol (36.3 ml) was added and then 98 % potassium tert-butoxide (101.9 g) was added between 40 °C to 55 °C.
**(xv)** This mixture was stirred for 15 min.
**(xvi)** Toluene and NMP were added to **Mixture A.**
**(xvii)** This mixture was heated up to 90 °C to 95 °C.
**(xviii)** This mixture was stirred for 4 h then cooled down to ambient temperature.
**(xix)** Water (325.5 ml) was added and a phase cut (organic phase up) was conducted.
**(xx)** Water (162.8 ml) was added and a phase cut (organic phase up) was conducted.
**(xxi)** Water (325.5 ml) was added and a phase cut (organic phase up) was conducted.
**(xxii)** Solvent was distilled out and ethanol (1672 ml) was added.
**(xxiii)** This mixture was loaded into a Parr reactor.
**(xxiv)** This mixture was flushed with nitrogen.
**(xxv)** 50 % wet 10 % Pd/C (3 g) was added.
**(xxiv)** The mixture was then flushed with hydrogen.
**(xxv)** The mixture was heated at 50 °C and pressurized up to 344737 Pa.
**(xxvi)** The mixture was stirred for 5 h.
**(xxvii)** The mixture was cooled down to ambient temperature.
**(xxviii)** The Parr reactor was depressurized down to atmospheric pressure and **hydrogen** was released from the mixture.
**(xxix)** The mixture was filtered on a pad of Hyflo Super Cel® (55.6 g).
**(xxx)** The mixture was rinsed with ethanol (167.2 ml)
**(xxxi)** Solvent was distilled from the mixture at atmospheric pressure.
**(xxxii)** 4-fluoro-3-methoxyphenol was distilled at 125 °C under 1 mbar.

### EXAMPLE 2.

**(i)** At ambient temperature, 2-butanone (1.5 I) and water (800 ml) were loaded in a flask;
**(ii)** Sodium iodide (56.9 g) and potassium carbonate (630 g) were added;
**(iii)** Tetrabutyl ammonium bromide (12.38 g) was added;
**(iv)** The mixture was heated to 50 °C;
**(v)** 3, 4-difluorophenol (500 g) diluted in 2-butanone (500 ml) was added slowly;
**(vi)** The mixture was stirred for 15 minutes;
**(vii)** 1-bromomethyl cyclopropane (622.5 g) was added;
**(viii)** The mixture was heated under reflux for 18 hours;
**(ix)** The reaction was cooled down to ambient temperature;
**(x)** Water (2 I) was added;
**(xi)** The reaction was stirred and a phase cut (organic phase up) was conducted;
**(xii)** The organic layer was washed with water (2 x 1 I) and a phase cut (organic phase up) was conducted;
**(xiii)** 2-butanone was distilled from the organic phase and toluene (870 ml) and NMP (183 ml) were added: **Mixture A;**
**(xiv)** Toluene (1.5 I) and NMP (366 ml) were loaded into a reactor;
**(xv)** The reaction mixture was stirred and heated to 40 °C;
**(xvi)** Methanol (158.2 ml) was added and then 98 % potassium tert-butoxide (446.55 g) was added between 40 °C to 55 °C;
**(xvii)** The reaction mixture was stirred for 15 min.;
**(xviii)** The reaction mixture was heated up to 80 °C to 85 °C;
**(xix) Mixture A** was added to the reaction mixture while keeping Tₘₐₛₛ < 95 °C;
**(xx)** The reaction mixture was stirred for 6 h then cooled down to ambient temperature;
**(xxi)** Water (1.2 I) was added and a phase cut (organic phase up) was conducted;
**(xxii)** Water (3 x 1.2 I) was added to the organic phase and a phase cut (organic phase up) was conducted;
**(xxiii)** 200 ml of toluene was distilled from the organic phase and toluene (0.32 I) was added;
**(xxiv)** The reaction was cooled down to 0 °C;
**(xxv)** 1, 3 dibromo-5, 5-dimethylhydantoin (453.2 g) diluted in tetrahydrofuran (1.3 I) was added over 1 h between 0 °C to 5 °C;
**(xxvi)** The reaction was stirred for 1 hour;
**(xxvii)** Water (906 ml) was added to the reaction.
(**xxviii**)A phase cut (organic phase up) was conducted;
**(xxix)** 2 M sodium hydroxide (0.9 I) was added to the organic phase and a phase cut (organic phase up) was conducted;
**(xxx**) Water (1 I) was added to the organic phase and a phase cut (organic phase up) was conducted;
**(xxxi**) Solvent was distilled from the organic phase and isopropyl alcohol (4.14 I) was added;
(xxxii)The mixture was heated under reflux, and then slowly cooled down to ambient temperature;
(xxxiii)The solid was filtered off and rinsed with isopropyl alcohol (2 x 400 ml); (**xxxiv**)The solid was dried at 35 °C under reduced pressure.

Processes of this invention are advantageous over current processes in that processes of this invention offers a straight access to prepare BCPMFMB through a minimum number of steps that are highly regioselective (e.g., more than 99% regioselectivity for some steps).

It is to be understood that the reactants and components referred to by chemical name or formula anywhere in the specification or claims hereof, whether referred to in the singular or plural, are identified as they exist prior to being combined with or coming into contact with another substance referred to by chemical name or chemical type (e.g., another reactant, a solvent, or etc.). It matters not what chemical changes, transformations and/or reactions, if any, take place in the resulting combination or solution or reaction medium as such changes, transformations and/or reactions are the natural result of bringing the specified reactants and/or components together under the conditions called for pursuant to this disclosure. Thus the reactants and components are identified as ingredients to be brought together in connection with performing a desired chemical reaction or in forming a combination to be used in conducting a desired reaction. Accordingly, even though the claims hereinafter may refer to substances, components and/or ingredients in the present tense ("comprises", "is", etc.), the reference is to the substance, component or ingredient as it existed at the time just before it was first contacted, combined, blended or mixed with one or more other substances, components and/or ingredients in accordance with the present disclosure. Whatever transformations, if any, which occur in situ as a reaction is conducted is what the claim is intended to cover. Thus the fact that a substance, component or ingredient may have lost its original identity through a chemical reaction or transformation during the course of contacting, combining, blending or mixing operations, if conducted in accordance with this disclosure and with the application of common sense and the ordinary skill of a chemist, is thus wholly immaterial for an accurate understanding and appreciation of the true meaning and substance of this disclosure and the claims thereof. As will be familiar to those skilled in the art, the terms "combined", "combining", and the like as used herein mean that the components that are "combined" or that one is "combining" are put into a container with each other. Likewise a "combination" of components means the components having been put together in a container.

While the present invention has been described in terms of one or more preferred embodiments, it is to be understood that other modifications may be made without departing from the scope of the invention, which is set forth in the claims below.

## Claims

1. A process comprising:
- reacting 3,4-difluorophenol in the presence of benzylbromide and potassium carbonate in 2-butanone, thereby yielding 4-(benzyloxy)-1,2-difluorobenzene;
- introducing methoxy moiety to 4-(benzyloxy)-1,2-difluorobenzene using a toluene/NMP mixture in the presence of potassium methoxide generated in situ from potassium tert-butoxide and methanol, thereby yielding 4-(benzyloxy)-1-fluoro-2-methoxybenzene;
- debenzylating 4-(benzyloxy)-1-fluoro-2-methoxybenzene under hydrogen pressure in the presence of palladium on charcoal; and
- distilling out 4-fluoro-3-methoxyphenol.

2. The process of claim 1 further comprising:
- O-alkylating the 4-fluoro-3-methoxyphenol with 1-bromomethyl cyclopropane, thereby yielding 3-methoxy-4-fluoro-1-cyclopropyl methoxybenzene; and
- brominating the 3-methoxy-4-fluoro-1-cyclopropyl methoxybenzene in the presence of 1,3-dibromo-5,5-dimethyl hydantoin or N-bromo succinimide, thereby yielding 1-bromo-2-(cyclopropyl methoxy)-5-fluoro-4-methoxybenzene.

3. The process of claim 1 wherein the toluene/NMP mixture is in about a 4:1 v/v mixture.

4. A process comprising:
- alkylating 3,4-difluorophenol, thereby yielding an O-alkylated phenol derivative which is 3,4-difluoro-1-cyclopropyl methoxybenzene;
- reacting the yielded O-alkylated phenol derivative with potassium methoxide in a toluene/NMP mixture, thereby yielding 3-methoxy-4-fluoro-1-cyclopropyl methoxybenzene;
- brominating the 3-methoxy-4-fluoro-1-cyclopropyl methoxy benzene in the presence of 1,3-dibromo-5,5-dimethylhydantoin or N-bromo succinimide; and
- crystallizing 1-bromo-2-(cyclopropyl methoxy)-5-fluoro-4-methoxybenzene from isopropyl alcohol or ethanol.

5. The process of claim 4 wherein the 0-alkylating of 3,4-difluorophenol comprises alkylating the 3,4-difluorophenol with 1-bromomethyl cyclopropane.

6. The process of claim 4 wherein the O-alkylating of 3,4-difluorophenol comprises alkylating the 3,4-difluorophenol with 1-chloromethyl cyclopropane.

7. The process of claim 4 wherein the toluene/NMP mixture is in about a 4:1 v/v mixture.

8. A process of claim 1 comprising:
- reacting 3,4-difluorophenol in the presence of benzylbromide and potassium carbonate in 2-butanone, thereby yielding 4-(benzyloxy)-1,2-difluorobenzene;
- introducing methoxy moiety to 4-(benzyloxy)-1,2-difluorobenzene using a toluene/NMP mixture in the presence of potassium methoxide generated in situ from potassium tert-butoxide and methanol, thereby yielding 4-(benzyloxy)-1-fluoro-2-methoxybenzene;
- debenzylating 4-(benzyloxy)-1-fluoro-2-methoxybenzene under hydrogen pressure in the presence of palladium on charcoal;
- distilling out 4-fluoro-3-methoxyphenol;
- O-alkylating the 4-fluoro-3-methoxyphenol with 1-bromomethyl cyclopropane, thereby yielding 3-methoxy-4-fluoro-1-cyclopropyl methoxybenzene; and
- brominating the 3-methoxy-4-fluoro-1-cyclopropyl methoxybenzene in the presence of 1,3-dibromo-5,5-dimethyl hydantoin or N-bromo succinimide, thereby yielding 1-bromo-2-(cyclopropyl methoxy)-5-fluoro-4-methoxybenzene.

## Patentansprüche

1. Verfahren, das umfasst:
- Umsetzen von 3,4-Difluorphenol in Gegenwart von Benzylbromid und Kaliumcarbonat in 2-Butanon, wodurch 4-(Benzyloxy)-1,2-difluorbenzol erhalten wird,
- Einführen von Methoxy-Anteil in 4-(Benzyloxy)-1,2-difluorbenzol unter Verwendung einer Toluol/NMP-Mischung in Gegenwart von Kaliummethoxid, das in situ aus Kalium-tert.butoxid und Methanol erzeugt wird, wodurch 4-(Benzyloxy)-1-fluor-2-methoxybenzol erhalten wird,
- Debenzylieren von 4-(Benzyloxy)-1-fluor-2-methoxybenzol unter Wasserstoffdruck in Gegenwart von Palladium auf Holzkohle, und
- Abdestillieren von 4-Fluor-3-methoxyphenol.

2. Verfahren nach Anspruch 1, das ferner umfasst:
- 0-Alkylierung von 4-Fluor-3-methoxyphenol mit 1-Brommethylcyclopropan, wodurch 3-Methoxy-4-fluor-1-cyclopropylmethoxybenzol erhalten wird, und
- Bromieren von 3-Methoxy-4-fluor-1-cyclopropylmethoxybenzol in Gegenwart von 1,3-Dibrom-5,5-dimethylhydantoin oder N-Bromsuccinimid, wodurch 1-Brom-2-(cyclopropylmethoxy)-5-fluor-4-methoxybenzol erhalten wird.

3. Verfahren nach Anspruch 1, bei dem die Toluol/NMP-Mischung eine etwa 4:1 vol./vol.-Mischung ist.

4. Verfahren, dass umfasst:
- Alkylieren von 3,4-Difluorphenol, wodurch ein O-alkyliertes Derivat erhalten wird, das 3,4-Difluor-1-cyclopropylmethoxybenzol ist,
- Umsetzen des erhaltenen O-alkylierten Derivats mit Kaliummethoxid in einer Toluol/NMP-Mischung, wodurch 3-Methoxy-4-fluor-1-cyclopropylmethoxybenzol erhalten wird,
- Bromieren von 3-Methoxy-4-fluor-1-cyclopropylmethoxybenzol in Gegenwart von 1,3-Dibrom-5,5-dimehtylhydantoin oder N-Bromsuccinimid, und
- Kristallisieren von 1-Brom-2-(cyclopropylmethoxy)-5-fluor-4-methoxybenzol.

5. Verfahren nach Anspruch 4, bei dem das 0-Alkylieren von 3,4-Difluorphenol das Alkylieren von 3,4-Difluorphenol mit 1-Brommethylcyclopropan umfasst.

6. Verfahren nach Anspruch 4, bei dem das 0-Alkylieren von 3,4-Difluorphenol das Alkylieren von 3,4-Difluorphenol mit 1-Chlormmethylcyclopropan umfasst.

7. Verfahren nach Anspruch 4, bei dem die Toluol/NMP-Mischung eine etwa 4:1 vol./vol.-Mischung ist.

8. Verfahren nach Anspruch 1, das umfasst:
- Umsetzen von 3,4-Difluorphenol in Gegenwart von Benzylbromid und Kaliumcarbonat in 2-Butanon, wodurch 4-(Benzyloxy)-1,2-difluorbenzol erhalten wird,
- Einführen von Methoxy-Anteil in 4-(Benzyloxy)-1,2-difluorbenzol unter Verwendung einer Toluol/NMP-Mischung in Gegenwart von Kaliummethoxid, das in situ aus Kalium-tert.butoxid und Methanol erzeugt wird, wodurch 4-(Benzyloxy)-1-fluor-2-methoxybenzol erhalten wird,
- Debenzylieren von 4-(Benzyloxy)-1-fluor-2-methoxybenzol unter Wasserstoffdruck in Gegenwart von Palladium auf Holzkohle,
- Abdestillieren von 4-Fluor-3-methoxyphenol,
- 0-Alkylieren von 4-Fluor-3-methoxyphenol mit 1-Brommethylcyclopropan, wodurch 3-Methoxy-4-fluor-1-cyclopropylmethoxybenzol erhalten wird,
- Bromieren von 3-Methoxy-4-fluor-1-cyclopropylmethoxybenzol in Gegenwart von 1,3-Dibrom-5,5-dimehtylhydantoin oder N-Bromsuccinimid, wodurch 1-Brom-2-(cyclopropylmethoxy)-5-fluor-4-methoxybenzol erhalten wird.

## Revendications

1. Processus comprenant :
- la réaction de 3,4-difluorophénol en présence de bromure de benzyle et de carbonate de potassium dans du 2-butanone, donnant de ce fait du 4-(benzyloxy)-1,2-difluorobenzène ;
- l'introduction de fraction de méthoxy dans du 4-(benzyloxy)-1,2-difluorobenzène en utilisant un mélange de toluène / NMP en présence de méthoxyde de potassium produit in situ à partir de tert-butoxyde de potassium et de méthanol, donnant de ce fait du 4-(benzyloxy)-1-fluoro-2-méthoxybenzène ;
- la débenzylation de 4-(benzyloxy)-1-fluoro-2-méthoxybenzène sous pression d'hydrogène en présence de palladium sur du charbon de bois ; et
- la distillation de 4-fluoro-3-méthoxyphénol.

2. Processus selon la revendication 1, comprenant en outre :
- l'O-alkylation du 4-fluoro-3-méthoxyphénol avec du 1-bromométhyle cyclopropane, donnant de ce fait du 3 méthoxy-4-fluoro-1-cyclopropyle méthoxybenzène ; et
- la bromuration du 3-méthoxy-4-fluoro-1-cyclopropyle méthoxybenzène en présence de 1,3-dibromo-5,5-diméthyle hydantoïne ou de N-bromo succinimide, donnant de ce fait du 1-bromo-2-(cyclopropyle méthoxy)-5-fluoro-4-méthoxybenzène.

3. Processus selon la revendication 1, dans lequel le mélange de toluène / NMP est dans un mélange d'environ 4:1 V/V.

4. Processus comprenant :
- l'alkylation de 3,4-difluorophénol, donnant de ce fait un dérivé de phénol O-alkylé qui est du 3,4-difluoro-1-cyclopropyle méthoxybenzène ;
- la réaction du dérivé de phénol O-alkylé obtenu avec du méthoxyde de potassium dans un mélange de toluène / NMP, donnant de ce fait du 3-méthoxy-4-fluoro-1-cyclopropyle méthoxybenzène ;
- la bromuration du 3-méthoxy-4-fluoro-1-cyclopropyle méthoxybenzène en présence de 1,3-dibromo-5,5-diméthylhydantoïne ou N-bromo succinimide ; et
- la cristallisation de 1-bromo-2-(cyclopropyle méthoxy)-5-fluoro-4-méthoxybenzène à partir d'alcool isopropylique ou d'éthanol.

5. Processus selon la revendication 4, dans lequel l'O-alkylation de 3,4-difluorophénol comprend l'alkylation du 3,4-difluorophénol avec du 1-bromométhyle cyclopropane.

6. Processus selon la revendication 4, dans lequel l'O-alkylation de 3,4-difluorophénol comprend l'alkylation du 3,4-difluorophénol avec du 1-chlorométhyle cyclopropane.

7. Processus selon la revendication 4, dans lequel le mélange de toluène / NMP est un mélange d'environ 4:1 V/V.

8. Processus selon la revendication 1 comprenant :
- la réaction de 3,4-difluorophénol en présence de bromure de benzyle et de carbonate de potassium dans du 2-butanone, donnant de ce fait du 4-(benzyloxy)-1,2-difluorobenzène ;
- l'introduction de fraction de méthoxy dans du 4-(benzyloxy)-1,2-difluorobenzène en utilisant un mélange de toluène / NMP en présence de méthoxyde de potassium produit in situ à partir de tert-butoxyde de potassium et de méthanol, donnant de ce fait du 4-(benzyloxy)-1-fluoro-2-méthoxybenzène ;
- la débenzylation de 4-(benzyloxy)-1-fluoro-2-méthoxybenzène sous pression d'hydrogène en présence de palladium sur du charbon de bois ;
- la distillation de 4-fluoro-3-méthoxyphénol ;
- l'O-alkylation du 4-fluoro-3-méthoxyphénol avec du 1-bromométhyle cyclopropane, donnant de ce fait du 3-méthoxy-4-fluoro-1-cyclopropyle méthoxybenzène ; et
- la bromuration de 3-méthoxy-4-fluoro-1-cyclopropyle méthoxybenzène en présence de 1,3-dibromo-5,5-diméthyle hydantoïne ou de N-bromo succinimide, donnant de ce fait du 1-bromo-2-(cyclopropyle méthoxy)-5-fluoro-4-méthoxybenzène.
